# EUROPÄISCHE PATENTSCHRIFT

(11) **EP 3 679 978 B1**
(45) Veröffentlichungstag und Bekanntmachung des Hinweises auf die Patenterteilung: **21.06.2023**
(21) Anmeldenummer: 19217483.7
(22) Anmeldetag: 18.12.2019
(51) Int. Cl.: A61M 39/22, A61M 39/28

(54) **KATHETERVENTIL UND VERSCHLUSSVORRICHTUNG FÜR EIN KATHETERVENTIL**
CATHETER VALVE AND CLOSURE DEVICE FOR A CATHETER VALVE
SOUPAPE DE CATHÉTER ET DISPOSITIF DE FERMETURE POUR UNE SOUPAPE DE CATHÉTER

(30) Priorität: 10.01.2019 DE 102019100509
(43) Veröffentlichungstag der Anmeldung: 15.07.2020
(73) Patentinhaber: Urovision Gesellschaft für Medizinischen Technologie Transfer mbH, 83043 Bad Aibling (DE)
(72) Erfinder: EDEN, Ingo, 81479 München (DE)
(74) Vertreter: Hofstetter, Schurack & Partner

(56) Entgegenhaltungen:
- EP-A1- 0 948 971
- US-A1- 2013 324 975

## Beschreibung

Die Erfindung betrifft ein Katheterventil gemäß dem Oberbegriff von Patentanspruch 1.

Ein derartiges Katheterventil kann beispielsweise eingesetzt werden um eine Durchflussmenge eines Mediums, beispielsweise Harn, welcher über einen Katheter aus einer Harnblase abgeführt werden kann, einzustellen. Zu diesem Zweck kann das Katheterventil mit einem Schlauchelement des Katheters gekoppelt werden. Über das Schlauchelement kann das Medium in einen Katheterventilinnenraum und dabei in ein Leitungselement des Katheterventils eingeführt werden. Anhand einer Verschlussvorrichtung des Katheterventils kann die Durchflussmenge durch das Leitungselement in der Regel eingestellt, also bedarfsgerecht unterbrochen und freigegeben werden. Die Verschlussvorrichtung kann meist mittels eines Stellelements manuell betätigt werden und dabei in eine Sperrstellung, in welcher die Durchflussmenge durch das Leitungselement unterbunden ist, sowie in eine Freigabestellung, in welcher die Durchflussmenge durch das Leitungselement freigegeben ist, bewegt werden.

Aus der CH 705 12 A2 ist ein selbstschließendes Katheterventil zur Verwendung in der Urologie bekannt. Das Katheterventil umfasst ein Gehäuse mit ovalem Querschnitt und mit einem konischen Schlauchaufsteckstutzen, auf den ein Katheterschlauch aufschiebbar ist. Zudem umfasst das Katheterventil ein Ventilschlauchstück, das das Gehäuse teilweise durchsetzt, ein Betätigungsorgan, mit dem das Ventil in seine Offenstellung bringbar ist und das auf ein Federelement wirkt und weitgehend innerhalb der Außenkontur im Gehäuse angeordnet ist. Das Betätigungsorgan ist in Richtung der Längsachse des Querschnittovals dergestalt bedienbar, dass bei Bedienung des Betätigungsorgans der Ventilschlauch seine Offenstellung einnimmt, und das Gehäuse eine Kammer umschließt, in der das Federelement durch Verformung des Ventilschlauchstücks dieses verschließt. Das Federelement ist eine Kippfeder, deren einer Schenkel an der Innenwandung der Kammer in ganzer Länge aufliegt. Die Schenkel des Federelements sind vollflächig und der flexible Schenkel weist auf beiden Längsseiten Schultern auf.

Die EP 0 948 971 A1 beschreibt ein Katheterventil, welches aus einem Gehäuse, einem elastisch verformbaren Rohr, das sich durch das Gehäuse erstreckt, Elementen zum Schließen und Öffnen des Rohrs und primären und sekundären Verbindungspunkten für einen Katheter besteht. Ein Verschluss des Katheterventils umfasst einen Betätigungshebel in dem Gehäuse, der eine primäre offene und eine sekundäre geschlossene Position aufweist. Eine Feder ist mit dem Hebel verbunden und hält ihn in der primären oder sekundären Position. Aus der US 2013/0324975 A1 ist ein Katheterventil gemäß den Merkmalen des Oberbegriffs des Anspruchs 1 bekannt.

Aufgabe der vorliegenden Erfindung ist es, ein Katheterventil sowie eine Verschlussvorrichtung der eingangs genannten Art mit einer verbesserten Handhabbarkeit zu schaffen.

Diese Aufgabe wird durch ein Katheterventil mit den Merkmalen des Patentanspruchs 1 gelöst. Vorteilhafte Weiterbildungen der Erfindung sind durch die Unteransprüche gegeben.

Die Erfindung betrifft ein Katheterventil zum Einstellen einer Durchflussmenge eines Mediums in einem Katheter. Die Durchflussmenge wird nachfolgend auch als "Durchfluss" bezeichnet. Das Katheterventil umfasst eine Ventilgehäusestruktur, welche einen Katheterventilinnenraum des Katheterventils zumindest bereichsweise begrenzt und wenigstens eine Bodenwand, die zur Auflage des Katheterventils ausgebildet ist, zumindest eine der Bodenwand gegenüberliegend angeordnete Oberwand sowie wenigstens eine, die Bodenwand und die Oberwand verbindende Seitenwand umfasst. Des Weiteren umfasst das Katheterventil zumindest ein Leitungselement zum Leiten des Mediums, welches sich zumindest im Wesentlichen in Längserstreckungsrichtung der Ventilgehäusestruktur durch den Katheterventilinnenraum erstreckt. Darüber hinaus umfasst das Katheterventil wenigstens eine Verschlussvorrichtung zum Sperren und Freigeben eines Durchflusses des Mediums durch das zumindest eine Leitungselement. Zudem umfasst das Katheterventil ein Stellelement, welches relativ zu der Ventilgehäusestruktur bewegbar gelagert ist und mittels welchem die wenigstens eine Verschlussvorrichtung zwischen einer Sperrstellung und einer Freigabestellung verstellbar ist. Unter dem Ausdruck, dass sich das Leitungselement "zumindest im Wesentlichen" in Längserstreckungsrichtung erstreckt ist vorliegend zu verstehen, dass sich das Leitungselement zumindest überwiegend in Längserstreckungsrichtung erstreckt. Mit anderen Worten erstreckt sich das Leitungselement weniger in Quererstreckungsrichtung und/oder Hocherstreckungsrichtung der Ventilgehäusestruktur sondern eher in Längserstreckungsrichtung. Das Katheterventil kann zur zumindest mittelbaren Koppelung mit einem Katheter-Leitungselement des Katheters ausgebildet sein. Das Katheter-Leitungselement, welches auch als Schlauchelement bezeichnet werden kann, kann zum Führen des Mediums ausgebildet sein. Das Stellelement kann vorzugsweise als Schieber ausgebildet sein.

Gemäß der Erfindung ist das Stellelement verschiebbar an oder in der zumindest einen Oberwand angeordnet. Dies ist von Vorteil, da durch diese Anordnung des Stellelements eine besonders gute Zugänglichkeit des Stellelements bei dessen Bedienung erzielt werden kann, was zu einer verbesserten Handhabbarkeit des Katheterventils beiträgt. So kann das Bewegen des Stellelement sogar mittels eines einzigen Fingers erfolgen, da durch die der Oberwand gegenüberliegende, vorzugsweise parallel gegenüberliegende, Bodenwand die Auflage besonders kippsicher erfolgen kann. Beispielsweise kann das Katheterventil an einem Oberschenkel eines Patienten aufliegen, sodass das Katheterventil beim Bewegen des Stellelements an dem Oberschenkel abgestützt werden kann, während das Stellelement durch den Finger bewegt wird. Darüber hinaus kann ein besonders platzsparendes, insbesondere eng an einem Körperteil anliegendes Tragen des Katheterventils durch eine Bedienperson erfolgen, wodurch das Katheterventil insgesamt besonders gut handhabbar sein kann. Denkbar ist prinzipiell auch, eine zumindest bereichsweise Beschichtung der Bodenwand mit einer Haftschicht, beispielsweise in Form einer Gummischicht, vorzusehen, sodass ein Verrutschen des Katheterventils in dessen Anlage am Oberschenkel während des Bewegens des Stellelements besonders weitgehend ausgeschlossen werden kann. Die Oberwand kann vorzugsweise eine einer Umgebung des Katheterventils zugewandte Haupterstreckungsfläche der Ventilgehäusestruktur aufweisen und insbesondere einen im Vergleich zu einer der Umgebung zugewandten Seitenwandfläche der zumindest einen, unmittelbar an die Oberwand angrenzenden Seitenwand größeren Flächeninhalt aufweisen, wodurch eine besonders gute Handhabbarkeit des Katheterventils gegeben ist.

Zudem umfasst die wenigstens eine Verschlussvorrichtung wenigstens zwei Federarmelemente, zwischen welchen ein Aufnahmebereich ausgebildet ist, in welchem zumindest ein Leitungsabschnitt des zumindest einen Leitungselements angeordnet ist und welche dazu ausgebildet sind, das zumindest eine Leitungselement zum Sperren des Durchflusses des Mediums in der Sperrstellung gemeinsam zusammenzudrücken. Dies ist von Vorteil, da durch das gemeinsame Zusammendrücken des Leitungselements dementsprechend beide Federarmelemente zum Sperren des Durchflusses durch das Leitungselement beitragen. Dadurch kann das Sperren des Durchflusses besonders zuverlässig erfolgen. Die wenigstens zwei Federarmelemente können vorzugsweise in einander entgegengesetzte Druckrichtungen auf das Leitungselement drücken, um den Durchfluss des Mediums durch das Leitungselement zu Sperren. Von Vorteil ist dabei, dass aufgrund der einander entgegengesetzten Druckrichtungen ein Kräftegleichgewicht zwischen den Federarmelementen ausgebildet werden kann, sodass eine Kraftübertragung an die Ventilgehäusestruktur vorzugsweise vollständig entfallen und die Ventilgehäusestruktur somit entlastet werden kann.

Die Haupterstreckungsfläche der Oberwand kann in besonders vorteilhafter Weise um einen Faktor mit einem Wert zwischen 1,1 und 100 größer sein als die Seitenwandfläche. Unter dem genannten Faktor sind dabei auch folgende Werte zu verstehen: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 sowie entsprechende Zwischenwerte. Dadurch kommt es zu einem besonders geringen sogenannten Auftragen des Katheterventils am Körper oder an einem Körperteil, beispielsweise an einem Oberschenkel, einer das Katheterventil und den Katheter tragenden Person, womit eine besonders gute Handhabbarkeit erreicht werden kann.

Zum Größenvergleich zwischen der Haupterstreckungsfläche und der Seitenwandfläche können Projektionen der Haupterstreckungsfläche und der Seitenwandfläche aus rechtwinklig zueinander versetzten Projektionsrichtungen miteinander verglichen werden, was insbesondere zweckmäßig ist, wenn die Haupterstreckungsfläche und/oder die Seitenwandfläche Unebenheiten aufweisen. Eine Projektion der Haupterstreckungsfläche kann durch Projizieren der Haupterstreckungsfläche in einer ersten Projektionsrichtung, welche beispielsweise entlang einer Hocherstreckungsrichtung der Ventilgehäusestruktur orientiert sein kann, erzeugt werden. Eine Projektion der Seitenwandfläche kann durch Projizieren der Seitenwandfläche in einer zweiten Projektionsrichtung, welche beispielsweise entlang einer senkrecht zur Hocherstreckungsrichtung orientierten Quererstreckungsrichtung der Ventilgehäusestruktur orientiert sein kann, erzeugt werden. Die Projektion der Haupterstreckungsfläche ist dann größer als die Projektion der Seitenwandfläche.

Die Haupterstreckungsfläche kann vorzugsweise flacher ausgebildet sein, als die Seitenwandfläche. Mit anderen Worten kann vorgesehen sein, dass die Haupterstreckungsfläche im Vergleich zur Seitenwandfläche insbesondere in Umfangsrichtung des Leitungselements weniger stark gewölbt ist, was ebenfalls zur verbesserten Handhabbarkeit des Katheterventils beiträgt. Unter dem Ausdruck weniger stark gewölbt ist dabei zu verstehen, dass die Haupterstreckungsfläche entweder im Vergleich zur Seitenwandfläche entweder keinerlei Wölbungen aufweist, oder im Vergleich zur Seitenwandfläche Wölbungen mit größerem Krümmungsradius aufweist.

Die in Umfangsrichtung des zumindest einen Leitungselements aneinander angrenzenden Wände (Bodenwand, Seitenwand bzw. Seitenwände, Oberwand) können gemeinsam einen zumindest im Wesentlichen rechteckigen Hohlquerschnitt der wenigstens einen Ventilgehäusestruktur bilden, wobei dieser (zumindest im Wesentlichen rechteckige) Hohlquerschnitt in einer senkrecht zur Längserstreckungsrichtung orientierten Ebene liegen kann. Die Wände können dabei einen Teil dieses Hohlquerschnitts bilden. Diese senkrecht zur Längserstreckungsrichtung orientierte Ebene kann beispielsweise durch die Quererstreckungsrichtung der Ventilgehäusestruktur sowie durch die Hocherstreckungsrichtung der Ventilgehäusestruktur aufgespannt sein. Unter dem Ausdruck "zumindest im Wesentlichen rechteckig" ist zu verstehen, dass der Hohlquerschnitt zumindest näherungsweise rechteckhohlprofilförmig sein kann, wobei der Hohlquerschnitt dabei beispielsweise Kantenverrundungen aufweisen kann. Aufgrund dieser Kantenverrundungen kann es zu Abweichungen von einer klassischen Rechteckhohlprofilform kommen, sodass der Hohlquerschnitt dementsprechend lediglich näherungsweise rechteckhohlprofilförmig sein kann. Durch diese Kantenverrundungen können zweckmäßigerweise scharfe Kanten zwischen den jeweiligen Begrenzungsflächen vermieden werden, sodass kein unangenehmes Eindrücken solcher Kanten am Körper bzw. Körperteil auftreten kann.

Die Ventilgehäusestruktur kann vorzugsweise zumindest teilweise aus thermoplastischem Polymer gebildet sein, wodurch die Ventilgehäusestruktur gleichsam besonders leicht und stabil ist.

In einer vorteilhaften Weiterbildung der Erfindung ist die wenigstens eine Verschlussvorrichtung durch lineares Verschieben des zumindest einen Stellelements in Längserstreckungsrichtung relativ zu der Ventilgehäusestruktur zwischen der Sperrstellung und der Freigabestellung verstellbar. Die Verschlussvorrichtung kann also durch lineares Verschieben des Stellelements in Längserstreckungsrichtung relativ zu der Ventilgehäusestruktur mittels des Stellelements zwischen der Sperrstellung und der Freigabestellung verstellbar sein. Von Vorteil ist dabei, dass ein derartiges, lineares Verschieben besonders intuitiv erfolgen kann, sodass beispielsweise eine sichere Handhabung auch bei einer Sehschwäche einer Bedienperson erfolgen kann. Bei dem linearen Verschieben kann eine Gleitbewegung des Stellelements an der Ventilgehäusestruktur, insbesondere an der Oberwand, entlang erfolgen. Das Stellelement kann manuell durch den Einsatz einer Hand oder alternativ durch den Einsatz mehrerer Hände linear verschoben werden. Vorzugsweise kann das Stellelement stufenlos relativ zu der Ventilgehäusestruktur linear verschiebbar an dieser gelagert sein. Durch die Verstellbarkeit des Stellelements in Längserstreckungsrichtung kann das Stellelement in einer Flussrichtung oder entgegen einer Flussrichtung des Mediums linear verschoben werden.

In einer weiteren vorteilhaften Weiterbildung der Erfindung ist das zumindest eine Stellelement wenigstens zwischen einer Freigabeposition, in welcher das zumindest eine Stellelement die wenigstens eine Verschlussvorrichtung in der Freigabestellung hält, und einer Sperrposition verschiebbar, in welcher das zumindest eine Stellelement die Sperrstellung der wenigstens einen Verschlussvorrichtung zulässt. Dies ist von Vorteil, da durch das Halten der Verschlussvorrichtung in der Freigabestellung anhand des Stellelements der Durchfluss bzw. die Durchflussmenge des Mediums dauerhaft aufrechterhalten werden kann. Die Sperrposition kann vorzugsweise durch ein auf dem Stellelement angeordnetes Schlosssymbol, insbesondere Vorhängeschlosssymbol, gekennzeichnet sein. Unter dem Ausdruck, dass "das Stellelement die Sperrstellung der Verschlussvorrichtung zulässt" ist vorliegend zu verstehen, dass "das Stellelement die Sperrstellung der Verschlussvorrichtung freigibt".

In einer weiteren vorteilhaften Weiterbildung der Erfindung bildet das zumindest eine Stellelement wenigstens in dessen Freigabeposition eine Rastverbindung mit der Ventilgehäusestruktur oder mit der wenigstens einen Verschlussvorrichtung. Dies ist von Vorteil, da der Durchfluss des Mediums durch die Rastverbindung auf besonders einfache Weise zuverlässig und dauerhaft aufrechterhalten werden kann, was ebenfalls zur verbesserten Handhabbarkeit beiträgt. In der Freigabeposition kann das zumindest eine Stellelement die wenigstens eine Verschlussvorrichtung also insgesamt dauerhaft in der Freigabestellung halten. Mit anderen Worten kann das zumindest eine Stellelement in dessen Freigabeposition die wenigstens eine Verschlussvorrichtung in einem Daueröffnungszustand halten. Dieser Daueröffnungszustand, also das dauerhafte Halten der wenigstens einen Verschlussvorrichtung in der Freigabestellung kann durch Verschieben des zumindest einen Stellelements von der Freigabeposition in die Sperrposition beendet werden. Das Stellelement kann in der Freigabeposition unter Ausbildung der Rastverbindung mit der Ventilgehäusestruktur oder mit der Verschlussvorrichtung verrasten. Bevorzugt kann die Rastverbindung hörbar und zusätzlich oder alternativ haptisch wahrnehmbar einstellbar sein, wodurch auch ohne optische Begutachtung des Stellelements bzw. des Katheterventils das Erreichen der Freigabeposition zuverlässig erkannt werden kann.

In einer weiteren vorteilhaften Weiterbildung der Erfindung verdeckt das zumindest eine Stellelement in dessen Sperrposition ein auf der Ventilgehäusestruktur angeordnetes Optikelement des Katheterventils und gibt in der Freigabeposition eine Sicht auf das Optikelement frei, wobei das Stellelement zusätzlich oder alternativ zu einer haptischen Positionsrückmeldung und zusätzlich oder alternativ zu einer auditiven Positionsrückmeldung zumindest beim Erreichen der Freigabeposition ausgebildet ist. Dies ist von Vorteil, da durch das Optikelement besonders schnell optisch erkannt werden kann, ob sich das Stellelement in der Sperrposition oder in der Freigabeposition befindet. Das Optikelement kann beispielsweise als farbige Markierung ausgebildet sein und dadurch besonders wenig Bauraum beanspruchen. Unter dem Ausdruck "Sicht auf das Optikelement" ist vorzugsweise eine Sicht auf das Optikelement zu verstehen, welche entlang einer senkrecht zur Oberwand bzw. senkrecht zu deren Haupterstreckungsfläche orientierten Blickrichtung erfolgen kann. Zusätzlich oder alternativ zu dem Optikelement kann die haptische Positionsrückmeldung infolge eines Einrastens des Stellelements, beispielsweise an der Ventilgehäusestruktur erfolgen. Die haptische Positionsrückmeldung kann beispielsweise in Form einer zumindest kurzzeitigen Vibration des Stellelements beim Erreichen der Freigabeposition und zusätzlich oder alternativ der Sperrposition erfolgen. Durch die haptische Positionsrückmeldung kann eine entsprechende Positionierung des Stellelements auch ohne Betrachtung des Katheterventils zuverlässig erfolgen, wodurch eine sichere Bedienung des Katheterventils durch Personen mit eingeschränktem Sehvermögen möglich ist. Darüber hinaus kann zusätzlich oder alternativ zu dem Optikelement und der haptischen Positionsrückmeldung die auditive, also hörbare, Positionsrückmeldung erfolgen. Die auditive Positionsrückmeldung kann beispielsweise in Form eines Klickgeräusches des Stellelements beim Erreichen der Freigabeposition und zusätzlich oder alternativ der Sperrposition erfolgen. Die haptische Positionsrückmeldung kann ebenfalls durch Einrasten des Stellelements bewirkt werden. Auch durch die auditive Positionsrückmeldung kann eine entsprechende Positionierung des Stellelements ohne Betrachtung des Katheterventils zuverlässig erfolgen, was eine sichere Bedienung des Katheterventils auch bei eingeschränktem Sehvermögen ermöglicht. Bevorzugt kann anhand des Optikelements, der haptischen Positionsrückmeldung und/oder der auditiven Positionsrückmeldung eine elektronikfreie (und damit nichtelektronische) Erkennung der Freigabeposition und zusätzlich oder alternativ der Sperrposition erfolgen. Dadurch kann in vorteilhafter Weise beispielsweise auf eine am Katheterventil vorgesehene, elektrische Energiequelle, insbesondere Batterie, zur Versorgung mit elektrischer Energie verzichtet werden.

In einer weiteren vorteilhaften Weiterbildung der Erfindung umfasst die wenigstens eine Verschlussvorrichtung zumindest ein Federelement, welches in der Sperrstellung eine jeweilige Verformungskraft auf die wenigstens zwei Federarmelemente ausübt, wodurch die wenigstens zwei Federarmelemente das zumindest eine Leitungselement zum Sperren des Durchflusses des Mediums in der Sperrstellung zusammendrücken. Dies ist von Vorteil, da dadurch, dass das zumindest eine Federelement in der Sperrstellung die jeweilige Verformungskraft auf die wenigstens zwei Federarmelemente ausübt, beide Federarmelemente anhand des zumindest einen Federelements verformt werden können, wodurch beide Federarmelemente das zumindest eine Leitungselement beispielsweise aus einander entgegengesetzten Richtungen zusammendrücken können. Dadurch, dass beide Federarmelemente anhand des zumindest einen Federelements verformbar sind, erfolgt bei jedem Federarmelement jeweils nur eine geringe Verformung über einen kleineren Verformungsweg als wenn nur ein einziges Federarmelement zum Zusammendrücken des Leitungselements vorgesehen wäre, welches entsprechend stärker verformt werden müsste. Das zumindest eine Federelement kann vorzugsweise als elastisch verformbarer Ring, insbesondere O-Ring, ausgebildet sein, wodurch das Federelement einen besonders einfachen Aufbau aufweist und insbesondere keinerlei Abstützung des Federelements an der Ventilgehäusestruktur erforderlich ist. Durch die Ausgestaltung als Ring kann das zumindest eine Federelement das zumindest eine Leitungselement in dessen Umfangsrichtung vollumfänglich und damit besonders platzsparend umschließen.

Das zumindest eine Leitungselement kann vorzugsweise wenigstens bereichsweise parallel zu den wenigstens zwei Federarmelementen orientiert sein. Dies stellt eine besonders platzsparende Anordnung des zumindest einen Leitungselements dar.

In einer weiteren vorteilhaften Weiterbildung der Erfindung umfasst die wenigstens eine Verschlussvorrichtung ein Gehäuseelement, welches mit der Ventilgehäusestruktur gekoppelt ist und welches den Katheterventilinnenraum des Katheterventils zumindest bereichsweise begrenzt. Dies ist von Vorteil, da die Verschlussvorrichtung somit eine besonders hohe Funktionalität aufweist und somit auch zum Abgrenzen und Verschließen des Katheterventilinnenraumes gegenüber der Umgebung des Katheterventils beiträgt. Das Gehäuseelement kann beispielsweise zur formschlüssigen Koppelung mit der Ventilgehäusestruktur ausgebildet sein. Dadurch kann das Gehäuseelement dementsprechend formschlüssig mit der Ventilgehäusestruktur gekoppelt sein. Bevorzugt kann das Gehäuseelement werkzeugfrei lösbar mit der Ventilgehäusestruktur koppelbar ausgebildet sein.

In einer weiteren vorteilhaften Weiterbildung der Erfindung sind die wenigstens zwei Federarmelemente einteilig mit dem Gehäuseelement verbunden. Dies ist von Vorteil, da hierdurch ein besonders einfacher Zusammenbau des Katheterventils ermöglicht ist. Die wenigstens zwei Federarmelemente können dabei integral mit dem Gehäuseelement, welches auch als Gehäuseteil bezeichnet werden kann, verbunden sein. Mittels des Gehäuseelements können die wenigstens zwei Federarmelemente besonders einfach in einem gegenseitigen Abstand zueinander gehalten sein.

In einer weiteren vorteilhaften Weiterbildung der Erfindung weist das Gehäuseelement eine Durchgangsöffnung zur Aufnahme des zumindest einen Leitungselements auf. Dies ist von Vorteil, da dies zu einer besonders hohen Funktionalität der Verschlussvorrichtung beiträgt. Vorzugsweise kann sich eine Mittelachse der Durchgangsöffnung zwischen den wenigstens zwei Federarmelementen erstrecken, sodass das Leitungselement anhand der Durchgangsöffnung zum Einstellen des Durchflusses besonders einfach zwischen den Federarmelementen positioniert werden kann.

Des Weiteren ist eine Verschlussvorrichtung für ein Katheterventil beschrieben. Die Verschlussvorrichtung umfasst wenigstens zwei Federarmelemente, zwischen welchen ein Aufnahmebereich zur Aufnahme zumindest eines Leitungsabschnitts zumindest eines Leitungselements des Katheterventils ausgebildet ist und welche dazu ausgebildet sind, das zumindest eine Leitungselement zum Sperren eines Durchflusses eines Mediums durch das zumindest eine Leitungselement in einer Sperrstellung der Verschlussvorrichtung gemeinsam zusammenzudrücken. Diese Verschlussvorrichtung ist besonders gut handhabbar und insbesondere für ein erfindungsgemäßes Katheterventil geeignet.

Die Verschlussvorrichtung umfasst zumindest ein Federelement, mittels welchem in der Sperrstellung eine jeweilige Verformungskraft auf die wenigstens zwei Federarmelemente ausübbar ist wodurch das zumindest eine Leitungselement zum Sperren des Durchflusses des Mediums in der Sperrstellung mittels der wenigstens zwei Federarmelemente zusammendrückbar ist.

Weitere Merkmale der Erfindung ergeben sich aus den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Dabei zeigt:
- Fig. 1: eine schematische Perspektivansicht auf ein Katheterventil; und
- Fig. 2: eine schematische Schnittdarstellung gemäß eines Schnittes parallel zu einer Längserstreckungsrichtung und einer Quererstreckungsrichtung einer Ventilgehäusestruktur des Katheterventils.

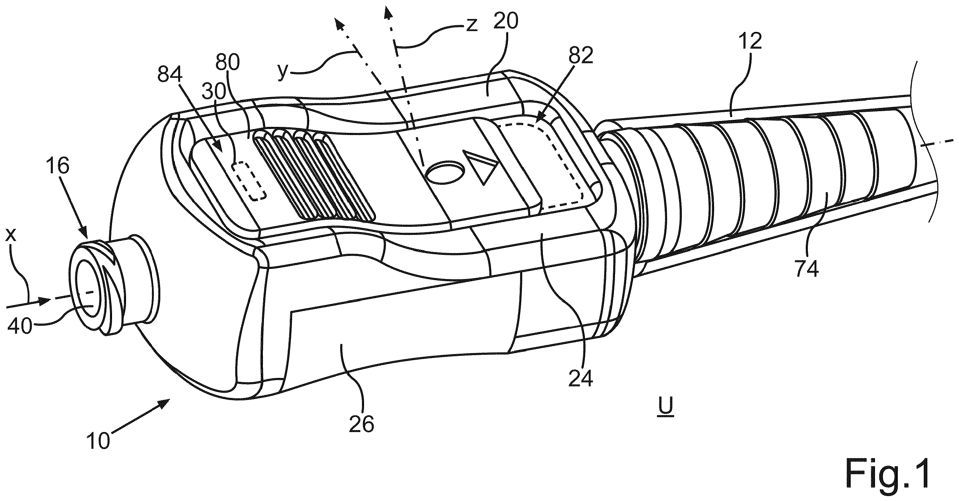

Fig. 1 zeigt in einer schematischen Perspektivansicht ein Katheterventil 10 zur Koppelung mit einem vorliegend lediglich teilweise gezeigten Katheter 12. In Fig. 1 ist von dem Katheter 12 lediglich ein Teilbereich eines Katheter-Leitungselements gezeigt.

Das Katheterventil 10 dient zum Einstellen einer Durchflussmenge eines mit dem Katheter 12 austauschbaren Mediums 14. Bei dem Medium 14 kann es sich beispielsweise um Harn handeln, welcher anhand des Katheters 12 aus einer Harnblase (nicht gezeigt) abgeführt werden kann. Die Durchflussmenge wird nachfolgend auch als Durchfluss bezeichnet.

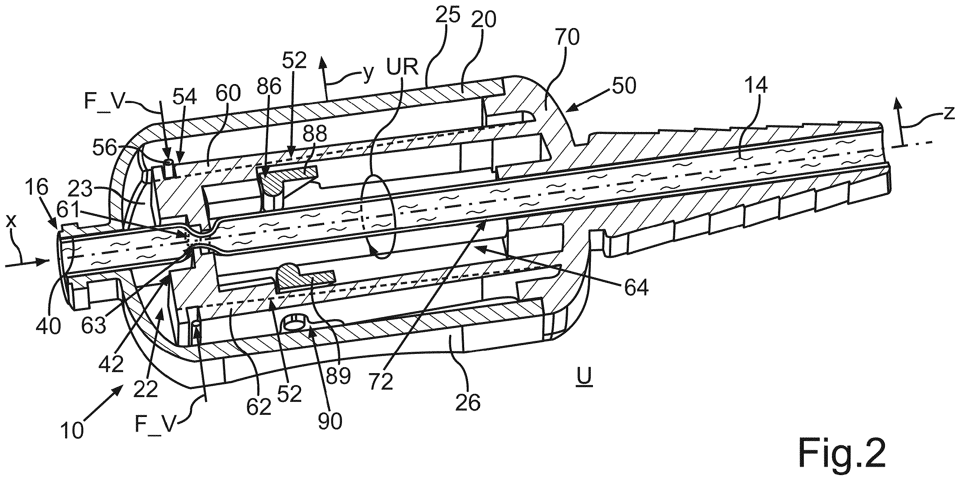

Das Katheterventil 10 umfasst eine Ventilgehäusestruktur 20, welche einen in Fig. 2 erkennbaren Katheterventilinnenraum 22 des Katheterventils 10 zumindest bereichsweise begrenzt. Das Katheterventil 10 umfasst des Weiteren einen Schnellverschluss 16, welcher sich entgegen einer Längserstreckungsrichtung x der Ventilgehäusestruktur 20 von dieser weg erstrecken kann, wie in Fig. 1 gezeigt ist. An dem Schnellverschluss 16 kann beispielsweise ein hier nicht weiter gezeigter Beutel zur Aufnahme des Mediums 14 angeschlossen werden.

Darüber hinaus umfasst das Katheterventil 10 ein elastisch verformbares Leitungselement 40 zum Leiten des Mediums 14 durch den Katheterventilinnenraum 22. Das Leitungselement 40 erstreckt sich vorliegend in Längserstreckungsrichtung x der Ventilgehäusestruktur 20 durch den gesamten Katheterventilinnenraum 22. Die Längserstreckungsrichtung x bildet zusammen mit einer Quererstreckungsrichtung y der Ventilgehäusestruktur 20 und einer Hocherstreckungsrichtung z der Ventilgehäusestruktur 20 ein Koordinatensystem.

Zudem umfasst das Katheterventil 10 eine Verschlussvorrichtung 50 zum Sperren des Durchflusses des Mediums 14 durch das Leitungselement 40 in einer Sperrstellung 52 der Verschlussvorrichtung 50 und zum Freigeben des Durchflusses des Mediums 14 durch das Leitungselement 40 in einer Freigabestellung 54 der Verschlussvorrichtung 50.

Des Weiteren umfasst das Katheterventil ein Stellelement 80, welches relativ zu der Ventilgehäusestruktur 20 bewegbar gelagert ist und mittels welchem die Verschlussvorrichtung 50 zwischen deren Sperrstellung 52 und deren Freigabestellung 54 verstellbar ist.

Die Ventilgehäusestruktur 20 weist mehrere, einer Umgebung U des Katheterventils 10 zugewandte und in Umfangsrichtung UR des Leitungselements 40 aneinander angrenzende Wände, nämlich eine Oberwand 24, welche einerseits an eine erste Seitenwand 25 und andererseits an eine zweite Seitenwand 26 angrenzt auf, wobei das Stellelement 80 an der Oberwand 24 angeordnet ist. Eine Bodenwand 23, die zur Auflage des Katheterventils 10 ausgebildet ist, grenzt in der Umfangrichtung UR einerseits an die erste Seitenwand 25 und andererseits an die zweite Seitenwand 26 an. Die Bodenwand 23 ist in Hocherstreckungsrichtung z der Ventilgehäusestruktur 20 gegenüberliegend zur Oberwand 24 angeordnet. Die Oberwand 24 weist eine der Umgebung U zugewandte Haupterstreckungsfläche auf, welche größer ist, als jeweilige der Umgebung U ebenfalls zugewandte Seitenwandflächen der Seitenwände 25, 26. Die Seitenwände grenzen dabei in der Umfangsrichtung UR an die Oberwand 24 an. Dementsprechend grenzen auch die jeweiligen Seitenwandflächen der Seitenwände 25, 26 an die Haupterstreckungsfläche der Oberwand 24 an.

Zum Größenvergleich zwischen der Haupterstreckungsfläche und den Seitenwandflächen können Projektionen der Haupterstreckungsfläche und Seitenwandflächen aus rechtwinklig zueinander versetzten Projektionsrichtungen miteinander verglichen werden. Eine Projektion der Haupterstreckungsfläche kann durch Projizieren der Haupterstreckungsfläche in einer ersten Projektionsrichtung welche entlang der Hocherstreckungsrichtung z orientiert sein kann, erzeugt werden. Jeweilige Projektionen der Seitenwandflächen können durch Projizieren der Seitenwandflächen entlang jeweiliger zweiter Projektionsrichtungen welche entlang der Quererstreckungsrichtung y orientiert sein können, erzeugt werden. Die Projektion der Haupterstreckungsfläche ist dann größer als die jeweiligen Projektionen der Seitenwandflächen.

Das Stellelement 80 ist verschiebbar an oder in der Oberwand 24 angeordnet, wodurch eine besonders gute Handhabbarkeit des Katheterventils 10 erzielt wird.

Die Verschlussvorrichtung 50 kann vorliegend durch lineares Verschieben des Stellelements 80 in Längserstreckungsrichtung x relativ zu der Ventilgehäusestruktur 20 zwischen der Sperrstellung 52 und der Freigabestellung 54 verstellt werden.

Die Verschlussvorrichtung 50 umfasst zwei Federarmelemente 60, 62, welche in Fig. 2 erkennbar sind. Diese Federarmelemente 60, 62 sind zumindest im Wesentlichen parallel zueinander orientiert. Zwischen den Federarmelementen 60, 62 ist ein Aufnahmebereich 64 ausgebildet, in welchem ein Leitungsabschnitt 42 des Leitungselements 40 angeordnet ist. Die Federarmelemente 60, 62 sind dazu ausgebildet, das Leitungselement 40 zum Sperren des Durchflusses des Mediums 14 in der Sperrstellung 52 gemeinsam zusammenzudrücken. Hierzu kann das erste Federarmelement 60 das Leitungselement 40 entgegen einer Pfeilrichtung eines die Quererstreckungsrichtung y verdeutlichenden Pfeils und das zweite Federarmelement 62 das Leitungselement 40 in der Pfeilrichtung dieses Pfeils zusammendrücken.

Das Stellelement 80 ist allgemein zwischen einer Freigabeposition 84, in welcher das Stellelement 80 die Verschlussvorrichtung 50 in der Freigabestellung 54 hält, und einer Sperrposition 82 verschiebbar, in welcher das Stellelement 80 die Sperrstellung 52 der Verschlussvorrichtung 50 zulässt. Die Sperrposition 82 des Stellelements 80 ist in Fig. 1 aus Gründen der Übersichtlichkeit lediglich schematisch gestrichelt angedeutet. In Fig. 1 ist hierzu erkennbar, dass das Stellelement 80 von dessen Freigabeposition 84 in Pfeilrichtung des die Längserstreckungsrichtung x verdeutlichenden Pfeils in die Sperrposition 82 verschoben werden kann.

Das Stellelement 80 verdeckt in dessen Sperrposition 82 ein auf der Ventilgehäusestruktur 20 angeordnetes Optikelement 30 des Katheterventils 10 und gibt in der Freigabeposition 84 eine Sicht auf das Optikelement 30 frei, wobei die Sicht auf das Optikelement 30 in Hocherstreckungsrichtung z und damit senkrecht auf die Haupterstreckungsfläche der Oberwand 24 erfolgt.

Das Stellelement 80 kann eine in den Katheterventilinnenraum 22 ragende Keilanordnung 86 aufweisen, welche vorliegend lediglich in Fig. 2 gezeigt ist. Die Keilanordnung kann, wie in Fig. 2 gezeigt, zwei voneinander beabstandete Keilelemente 88, 89 aufweisen. Die Keilelemente 88, 89 können auch als Keile bezeichnet werden.

Das Stellelement 80 kann in dessen Freigabeposition 84 eine Rastverbindung 90 mit der Ventilgehäusestruktur 20 oder mit der Verschlussvorrichtung 50 bilden. Vorliegend kann die Rastverbindung 90 durch Einrasten der Keilelemente 88, 89 in die Federarmelemente 60, 62 gebildet werden. Dabei rastet das Keilelement 88 an dem ersten Federarmelement 60 und das Keilelement 89 an dem zweiten Federarmelement 62 ein. Beim Einrasten kann anhand des Stellelements 80 eine haptische und/oder auditive Rückmeldung an eine das Stellelement 80 betätigende Bedienperson erfolgen. So können beim Einrasten eine durch die Bedienperson spürbare Vibration bzw. ein durch die Bedienperson hörbares Klickgeräusch am Stellelement 80 entstehen.

Die Keilelemente 88, 89 drücken die Federarmelemente 60, 62 in der Freigabeposition 84 des Stellelements 80 in Quererstreckungsrichtung y zumindest bereichsweise auseinander, wodurch die Verschlussvorrichtung 50 in der Freigabestellung 54 gehalten wird.

Die Verschlussvorrichtung 50 umfasst ein Federelement 56, welches in der Sperrstellung 52 eine jeweilige Verformungskraft F_V auf die beiden Federarmelemente 60, 62 ausübt, wodurch die Federarmelemente 60, 62 das Leitungselement 40 zum Sperren des Durchflusses des Mediums 14 in der Sperrstellung 52 zusammendrücken.

Um eine besonders gute Abdichtung des Leitungselements 40 zu erreichen, weist das erste Federarmelement 60 einen ersten Stufenvorsprung 61 und das zweite Federarmelement 62 einen zweiten Stufenvorsprung 63 auf. Unter dem Ausdruck "Stufenvorsprung" ist vorliegend ein gestufter, also treppenartig ausgebildeter Vorsprung zu verstehen. Die Stufenvorsprünge 61, 63 ragen vorliegend entlang der Quererstreckungsrichtung y von dem ersten Federarmelement 60 bzw. von dem zweiten Federarmelement 62 ab. Vorzugsweise weisen die Stufenvorsprünge 61, 63 zueinander komplementäre Stufen auf, wie in Fig. 2 erkennbar ist, wodurch eine besonders gute Dichtwirkung erzielt werden kann.

Beim Bewegen in die Sperrstellung 52 drückt das vorliegend als O-Ring ausgebildete Federelement 56, welches das Leitungselement 40 in dessen Umfangsrichtung UR umgibt, die beiden Federarmelemente 60, 62 durch das Ausüben der jeweiligen Verformungskraft F_V im Bereich der Stufenvorsprünge 61, 63 zusammen. Dadurch nähern sich die Stufenvorsprünge 61, 63 entlang der Quererstreckungsrichtung y einander an und das Leitungselement 40 wird schließlich (an dem Leitungsabschnitt 42) durch die Stufenvorsprünge 61, 63 zusammengedrückt, wodurch der Durchfluss des Mediums 14 durch das Leitungselement 40 bzw. durch den Leitungsabschnitt 42 unterbunden wird. Die Sperrstellung 52 ist vorliegend nicht explizit gezeigt, jedoch zumindest durch eine jeweilige gestrichelte Darstellung jeweiliger Kanten der jeweiligen Federarmelemente 60, 62 zumindest angedeutet. Diese Kanten der jeweiligen Federarmelemente 60, 62 sind jeweils entlang der Quererstreckungsrichtung y den jeweiligen Stufenvorsprüngen 61, 63 gegenüberliegend angeordnet.

Wird das Stellelement 80 von der Freigabeposition 84 in die Sperrposition 82 bewegt (verschoben) so gleiten die der Verformungskraft F_V in der Freigabestellung 54 bzw. der Freigabeposition 84 entgegen wirkenden Keilelemente 88, 89 an den jeweiligen Federarmelemente in 60, 62 in Längserstreckungsrichtung x entlang. Die Keilelemente 88, 89 entfernen sich dabei in Längserstreckungsrichtung x von den Stufenvorsprüngen 61, 63. Bei diesem Gleiten der Keilelemente 88, 89 in Pfeilrichtung der Längserstreckungsrichtung x werden die Federarmelemente 60, 62 mittels des Federelements 56 (durch Ausübung der jeweiligen Verformungskraft F_V) sukzessive im Bereich der Stufenvorsprung 61, 63 einander angenähert bis schließlich die Sperrstellung 52 erreicht ist.

Die Verschlussvorrichtung 50 umfasst zudem ein Gehäuseelement 70, welches mit der Ventilgehäusestruktur 20 gekoppelt ist und welches den Katheterventilinnenraum 22 des Katheterventils 10 zumindest bereichsweise begrenzt. Das Gehäuseelement 70 ist an jeweiligen, in Längserstreckungsrichtung x den Stufenvorsprüngen 61, 63 gegenüberliegenden Federarmelementenden der jeweiligen Federarmelemente 60, 62 angeordnet. Die Federarmelemente 60, 62 sind dabei einteilig mit dem Gehäuseelement 70 verbunden.

Das Gehäuseelement 70 weist eine Durchgangsöffnung 72 zur Aufnahme des zumindest einen Leitungselements 40 auf. Die Durchgangsöffnung 72 erstreckt sich durch einen konisch geformten Anschlussstutzen 74, an welchem das Katheter-Leitungselement des Katheters 12 aufgesteckt ist. Über die Durchgangsöffnung 72 kann das Medium 14 entgegen der Pfeilrichtung des die Längserstreckungsrichtung x verdeutlichenden Pfeils durch den Katheterventilinnenraum 22 geführt werden, wobei das Medium 14 im Bereich des Schnellverschlusses 16 aus dem Katheterventilinnenraum 22 und damit aus dem Katheterventil 10 austreten kann. Zusammenfassend kann durch die Positionierung des Stellelements 80, welches auch als Steuerelement bezeichnet werden kann, auf oder an der Oberwand 24, welche mit deren Haupterstreckungsfläche eine flache Seite der Ventilgehäusestruktur 20 darstellt, eine besonders vorteilhafte Handhabung des Katheterventils 10 erzielt werden. Diese Positionierung ermöglicht eine Verwendung des Katheterventils 10 in einer flachen Anlage an einem Körperteil, beispielsweise einem Oberschenkel einer das Katheterventil 10 tragenden Person, also beispielsweise eines Patienten, wobei das Katheterventil 10 lediglich in geringem Maße aufträgt. Die Positionierung des Stellelements 80 auf oder an der Oberwand 24, also beispielsweise an der Haupterstreckungsfläche (flache Seite) wird durch die Verschlussvorrichtung 50, welche mit dem Federelement 56 ein Feder-Spannsystem bildet, vereinfacht ermöglicht. Dieses Feder-Spannsystem umfasst die integralen, außerhalb des Leitungselements 40 angeordneten Federarmelemente 60, 62, welche auch als Federarme bezeichnet werden können, und welche mittels des umlaufenden Federelements 56 (O-Ring) initial in der Sperrstellung 52 gehalten werden können. Die Sperrstellung 52 kann auch als Geschlossen-Stellung bezeichnet werden. In der Sperrstellung 52 drücken die Stufenvorsprünge 61, 63, welche auch als jeweilige Federarmfortsätze bezeichnet werden können, das in dem Aufnahmebereich 64 angeordnete Leitungselement 40, welches als Silikonschlauch ausgebildet sein kann, zusammen. Um den Durchfluss des Mediums 14 durch das Katheterventil 10 zu ermöglichen, also das Katheterventil 10 zu öffnen, wird das Stellelement 80 (Steuerelement) gegen die Pfeilrichtung der Längserstreckungsrichtung x und damit beispielsweise in Flussrichtung des Mediums 14 zurückgezogen bzw. geschoben. Durch eine Keilwirkung zwischen den Federarmelementen 60, 62 und den Keilelementen 88, 89 der Keilanordnung 86 des Stellelements 80 werden die Federarmelemente 60, 62 beim Bewegen des Stellelements 80 von der Sperrposition 82 in die Freigabeposition 84 entgegen der Verformungskraft F_V das Federelement 56 entlang der Quererstreckungsrichtung y aufgedrückt und damit entlang der Quererstreckungsrichtung y im Bereich der Stufenvorsprung 61, 63 auseinandergedrückt, wodurch das Katheterventil 10 geöffnet und der Durchfluss des Mediums 14 durch das Leitungselement 40 freigegeben wird. An zumindest einem der Keilelemente 88, 89 kann ein integraler Rasthaken ausgebildet sein, welcher mit zumindest einem der Federarmelemente 60, 62 oder alternativ mit der Ventilgehäusestruktur 20 in der Freigabeposition 84 des Stellelements 80 sowie in der Freigabestellung 54 der Verschlussvorrichtung 50 verhaken bzw. verrasten kann, wodurch eine dauerhafte Durchströmung des Leitungselements 40 zugelassen ist. Zum Schließen des Katheterventils 10 wird das Stellelement 80 in Pfeilrichtung der Längserstreckungsrichtung x und damit beispielsweise entgegen der Flussrichtung des Mediums 14 von der Freigabeposition 84 in die Sperrposition 82 bewegt, wobei die Rastverbindung 90 (und damit der integrale Rasthaken) gelöst und die Federarmelemente 60, 62 durch die Verformungskraft F_V mittels des Federelements 56 zusammengedrückt werden, wodurch die Verschlussvorrichtung 50 von der Freigabestellung 54 in die Sperrstellung 52 versetzt werden kann. Das Katheterventil 10 kann also mit anderen Worten durch die Verformungskraft F_V, welche auch als Rückstellkraft bezeichnet werden kann, selbstständig schließen. Besonders hervorzuheben ist, dass das Katheterventil 10 - wie in Fig. 1 und Fig 2 erkennbar ist - lediglich aus fünf Einzelteilen bestehen kann, nämlich aus der Ventilgehäusestruktur 20, dem Leitungselement 40, der Verschlussvorrichtung 50 dem Federelement 56 und dem Stellelement 80. Dadurch ist eine besonders einfache mechanische Montage des Katheterventils 10 durch Zusammenfügen dieser Einzelteile ermöglicht. Für die Montage sind keine speziellen Fügeverfahren notwendig. Darüber hinaus kann durch die flache, ergonomische Form, also aufgrund dessen, dass die Haupterstreckungsfläche der Oberwand 24 vorliegend größer ist als die jeweiligen Seitenwandflächen der Seitenwände 25, 26 eine einfache Bedienung erfolgen, was insbesondere für Personen mit einem Handicap von Vorteil ist.

## Patentansprüche

1. Katheterventil (10) zum Einstellen einer Durchflussmenge eines Mediums (14) in einem Katheter (12), umfassend zumindest
- eine Ventilgehäusestruktur (20), welche einen Katheterventilinnenraum (22) des Katheterventils (10) zumindest bereichsweise begrenzt und wenigstens eine Bodenwand (23), die zur Auflage des Katheterventils (10) ausgebildet ist, zumindest eine der Bodenwand (23) gegenüberliegend angeordnete Oberwand (24) sowie wenigstens eine, die Bodenwand (23) und die Oberwand (24) verbindende Seitenwand (26) umfasst;
- zumindest ein Leitungselement (40) zum Leiten des Mediums (14), welches sich zumindest im Wesentlichen in Längserstreckungsrichtung (x) der Ventilgehäusestruktur (20) durch den Katheterventilinnenraum (22) erstreckt;
- wenigstens eine Verschlussvorrichtung (50) zum Sperren und Freigeben eines Durchflusses des Mediums (14) durch das zumindest eine Leitungselement (40); und
- ein Stellelement (80), welches relativ zu der Ventilgehäusestruktur (20) bewegbar gelagert ist und mittels welchem die wenigstens eine Verschlussvorrichtung (50) zwischen einer Sperrstellung (52) und einer Freigabestellung (54) verstellbar ist, wobei das Stellelement (80) verschiebbar an oder in der zumindest einen Oberwand (24) angeordnet ist,
**dadurch gekennzeichnet, dass**
die wenigstens eine Verschlussvorrichtung (50) wenigstens zwei Federarmelemente (60, 62) umfasst, zwischen welchen ein Aufnahmebereich (64) ausgebildet ist, in welchem zumindest ein Leitungsabschnitt (42) des zumindest einen Leitungselements (40) angeordnet ist und welche dazu ausgebildet sind, das zumindest eine Leitungselement (40) zum Sperren des Durchflusses des Mediums (14) in der Sperrstellung (52) gemeinsam zusammenzudrücken.

2. Katheterventil (10) nach Anspruch 1,
**dadurch gekennzeichnet, dass**
die wenigstens eine Verschlussvorrichtung (50) durch lineares Verschieben des zumindest einen Stellelements (80) in Längserstreckungsrichtung (x) relativ zu der Ventilgehäusestruktur (20) zwischen der Sperrstellung (52) und der Freigabestellung (54) verstellbar ist.

3. Katheterventil (10) nach Anspruch 1 oder 2,
**dadurch gekennzeichnet, dass**
das zumindest eine Stellelement (80) wenigstens zwischen einer Freigabeposition (84), in welcher das zumindest eine Stellelement (80) die wenigstens eine Verschlussvorrichtung (50) in der Freigabestellung (54) hält, und einer Sperrposition (82) verschiebbar ist, in welcher das zumindest eine Stellelement (80) die Sperrstellung (52) der wenigstens einen Verschlussvorrichtung (50) zulässt.

4. Katheterventil (10) nach Anspruch 3,
**dadurch gekennzeichnet, dass**
das zumindest eine Stellelement (80) wenigstens in dessen Freigabeposition (84) eine Rastverbindung (90) mit der Ventilgehäusestruktur (20) oder mit der wenigstens einen Verschlussvorrichtung (50) bildet.

5. Katheterventil (10) nach Anspruch 3 oder 4,
**dadurch gekennzeichnet, dass**
das zumindest eine Stellelement (80) in dessen Sperrposition (82) ein auf der Ventilgehäusestruktur (20) angeordnetes Optikelement (30) des Katheterventils (10) verdeckt und in der Freigabeposition (84) eine Sicht auf das Optikelement (30) freigibt und/oder dass das Stellelement (80) zu einer haptischen Positionsrückmeldung und/oder einer auditiven Positionsrückmeldung zumindest beim Erreichen der Freigabeposition (84) ausgebildet ist.

6. Katheterventil (10) nach Anspruch 1,
**dadurch gekennzeichnet, dass**
die wenigstens eine Verschlussvorrichtung (50) zumindest ein Federelement (56) umfasst, welches in der Sperrstellung (52) eine jeweilige Verformungskraft (F_V) auf die wenigstens zwei Federarmelemente (60, 62) ausübt, wodurch die wenigstens zwei Federarmelemente (60, 62) das zumindest eine Leitungselement (40) zum Sperren des Durchflusses des Mediums (14) in der Sperrstellung (52) zusammendrücken.

7. Katheterventil (10) nach einem der vorhergehenden Ansprüche,
**dadurch gekennzeichnet, dass**
die wenigstens eine Verschlussvorrichtung (50) ein Gehäuseelement (70) umfasst, welches mit der Ventilgehäusestruktur (20) gekoppelt ist und welches den Katheterventilinnenraum (22) des Katheterventils (10) zumindest bereichsweise begrenzt.

8. Katheterventil (10) nach Anspruch 7 in dessen Rückbezug auf Anspruch 1 oder 6,
**dadurch gekennzeichnet, dass**
die wenigstens zwei Federarmelemente (60, 62) einteilig mit dem Gehäuseelement (70) verbunden sind.

9. Katheterventil (10) nach Anspruch 7 oder 8,
**dadurch gekennzeichnet, dass**
das Gehäuseelement (70) eine Durchgangsöffnung (72) zur Aufnahme des zumindest einen Leitungselements (40) aufweist.

## Claims

1. A catheter valve (10) for adjusting a flow rate of a medium (14) in a catheter (12), comprising at least
- a valve housing structure (20), which delimits a catheter valve interior (22) of the catheter valve (10) at least in certain areas, and includes at least one bottom wall (23), which is formed for support of the catheter valve (10), at least one top wall (24) arranged opposing the bottom wall (23) as well as at least one sidewall (26) connecting the bottom wall (23) and the top wall (24);
- at least one line element (40) for conducting the medium (14), which extends through the catheter valve interior (22) at least substantially in longitudinal extension direction (x) of the valve housing structure (20);
- at least one closure device (50) for blocking and unblocking a flow of the medium (14) through the at least one line element (40); and
- an adjusting element (80), which is mounted movable in relation to the valve housing structure (20) and by means of which the at least one closure device (50) is adjustable between a blocking position (52) and an unblocking position (54), wherein the adjusting element (80) is arranged displaceable on or in the at least one top wall (24),
**characterized in that**
the at least one closure device (50) includes at least two spring arm elements (60, 62), between which a receiving area (64) is formed, in which at least a line section (42) of the at least one line element (40) is arranged, and which are formed to commonly compress the at least one line element (40) for blocking the flow of the medium (14) in the blocking position (52).

2. The catheter valve (10) according to claim 1,
**characterized in that**
the at least one closure device (50) is adjustable between the blocking position (52) and the unblocking position (54) by linearly displacing the at least one adjusting element (80) in longitudinal extension direction (x) in relation to the valve housing structure (20).

3. The catheter valve (10) according to claim 1 or 2,
**characterized in that**
the at least one adjusting element (80) is displaceable at least between an unblocking position (84), in which the at least one adjusting element (80) retains the at least one closure device (50) in the unblocking position (54), and a blocking position (82), in which the at least one adjusting element (80) allows the blocking position (52) of the at least one closure device (50).

4. The catheter valve (10) according to claim 3,
**characterized in that**
the at least one adjusting element (80), at least in the unblocking position (84) thereof, forms a locking connection (90) with the valve housing structure (20) or with the at least one closure device (50).

5. The catheter valve (10) according to claim 3 or 4,
**characterized in that**
the at least one adjusting element (80), in the blocking position (82) thereof, covers an optical element (30) of the catheter valve (10) arranged on the valve housing structure (20) and unblocks a view to the optical element (30) in the unblocking position (84), and/or that the adjusting element (80) is formed for a haptic position feedback and/or an auditive position feedback at least upon reaching the unblocking position (84).

6. The catheter valve (10) according to claim 1,
**characterized in that**
the at least one closure device (50) includes at least one spring element (56), which exerts a respective deformation force (F_V) on the at least two spring arm elements (60, 62) in the blocking position (52), whereby the at least two spring arm elements (60, 62) compress the at least one line element (40) for blocking the flow of the medium (14) in the blocking position (52).

7. The catheter valve (10) according to any one of the preceding claims,
**characterized in that**
the at least one closure device (50) includes a housing element (70), which is coupled to the valve housing structure (20) and which delimits the catheter valve interior (22) of the catheter valve (10) at least in certain areas.

8. The catheter valve (10) according to claim 7 in relation thereof to claim 1 or 6,
**characterized in that**
the at least two spring arm elements (60, 62) are integrally connected to the housing element (70).

9. The catheter valve (10) according to claim 7 or 8,
**characterized in that**
the housing element (70) comprises a passage opening (72) for receiving the at least one line element (40).

## Revendications

1. Valve de cathéter (10) pour le réglage d'un débit d'un fluide (14) dans un cathéter (12), comprenant au moins :
- une structure de boîtier de valve (20) qui délimite, au moins dans certaines zones, un espace intérieur de valve de cathéter (22) de la valve de cathéter (10) et qui comprend au moins une paroi de fond (23) conçue pour supporter la valve de cathéter (10), au moins une paroi supérieure (24) agencée en face de la paroi de fond (23) ainsi qu'au moins une paroi latérale (26) reliant la paroi de fond (23) et la paroi supérieure (24),
- au moins un élément de guidage (40) pour guider le fluide (14), lequel élément de guidage s'étend au moins sensiblement dans une direction d'extension longitudinale (x) de la structure de boîtier de valve (20) à travers l'espace intérieur de valve de cathéter (22) ;
- au moins un dispositif de fermeture (50) pour bloquer et libérer un écoulement du fluide (14) à travers le au moins un élément de guidage (40) ; et
- un élément de réglage (80) qui est monté de manière mobile par rapport à la structure de boîtier de valve (20) et au moyen duquel le au moins un dispositif de fermeture (50) peut être réglé entre une position de blocage (52) et une position de libération (54), l'élément de réglage (80) étant agencé de manière mobile sur ou dans la au moins une surface supérieure (24),
**caractérisée en ce que**
le au moins un dispositif de fermeture (50) comprend au moins deux éléments de bras à ressort (60, 62) entre lesquels est formée une zone de réception (64) dans laquelle au moins une partie de guidage (42) du au moins un élément de guidage (40) est agencée, et qui sont conçus pour comprimer ensemble le au moins un élément de guidage (40) afin de bloquer l'écoulement du fluide (14) dans la position de blocage (52).

2. Valve de cathéter (10) selon la revendication 1,
**caractérisée en ce que**
le au moins un dispositif de fermeture (50) peut être réglé entre la position de blocage (52) et la position de libération (54) par un déplacement linéaire du au moins un élément de réglage (80) dans la direction d'extension longitudinale (x) par rapport à la structure de boîtier de valve (20).

3. Valve de cathéter (10) selon la revendication 1 ou 2,
**caractérisée en ce que**
le au moins un élément de réglage (80) peut être déplacé au moins entre une position de libération (84), dans laquelle le au moins un élément de réglage (80) maintient le au moins un dispositif de fermeture (50) dans la position de libération (54), et une position de blocage (82), dans laquelle le au moins un élément de réglage (80) permet la position de blocage (52) du au moins un dispositif de fermeture (50).

4. Valve de cathéter (10) selon la revendication 3,
**caractérisée en ce que**
le au moins un élément de réglage (80) forme, au moins dans sa position de libération (84), un assemblage par encliquetage (90) avec la structure de boîtier de valve (20) ou avec le au moins un dispositif de fermeture (50).

5. Valve de cathéter (10) selon la revendication 3 ou 4,
**caractérisée en ce que**
le au moins un élément de réglage (80) masque, dans sa position de blocage (82), un élément optique (30) de la valve de cathéter (10) agencé sur la structure de boîtier de valve (20) et permet, dans la position de libération (84), une vue de l'élément optique (30) et/ou **en ce que** l'élément de réglage (80) est conçu pour un retour d'information de position haptique et/ou un retour d'information de position auditif au moins lorsque la position de libération (84) est atteinte.

6. Valve de cathéter (10) selon la revendication 1,
**caractérisée en ce que**
le au moins un dispositif de fermeture (50) comprend au moins un élément à ressort (56) qui exerce, dans la position de blocage (52), une force de déformation (F_V) respective sur les au moins deux éléments de bras à ressort (60, 62), en sorte que les au moins deux éléments de bras à ressort (60, 62) compriment le au moins un élément de guidage (40) afin de bloquer l'écoulement du fluide (14) dans la position de blocage (52).

7. Valve de cathéter (10) selon l'une des revendications précédentes,
**caractérisée en ce que**
le au moins un dispositif de fermeture (50) comprend un élément de boîtier (70) qui est couplé à la structure de boîtier de valve (20) et qui délimite, au moins dans certaines zones, l'espace intérieur de valve de cathéter (22) de la valve de cathéter (10).

8. Valve de cathéter (10) selon la revendication 7 lorsque dépendante de la revendication 1 ou 6,
**caractérisée en ce que**
les au moins deux éléments de bras à ressort (60, 62) sont reliés à l'élément de boîtier (70) de manière solidaire.

9. Valve de cathéter (10) selon la revendication 7 ou 8,
**caractérisée en ce que**
l'élément de boîtier (70) comporte un orifice de passage (72) pour recevoir le au moins un élément de guidage (40).
